# EUROPEAN PATENT APPLICATION

(11) **EP 1 172 445 A1**
(43) Date of publication of application: **16.01.2002**
(21) Application number: 00115268.5
(22) Date of filing: 14.07.2000
(51) Int. Cl.: C12Q 1/68, B01L 3/14

(54) **A method for direct genetic analysis of target cells by using fluorescence probes**

(71) Applicant: Praenadia GmbH, 48155 Münster (DE)
(72) Inventor: Wiebusch, Heiko, Dr., 48161 Münster (DE); Schmitt-John, Thomas, Dr., 33803 Steinhagen (DE); Weidner, Jürgen, Dr., 48143 Münster (DE)
(74) Representative: König, Reimar, Dr.-Ing.

(57) **Abstract**

A method enables the identification of target cells from non-target cells comprising the step of in situ hybridising a target sequence in a target cell with a labelled sequence and cell identification of the target cell by the aid of detecting the hybridised labelled sequence by flow cytometry.

## Description

This invention pertains to a method of separating target cells from non-target cells, especially fetal cells from maternal blood cells for prenatal diagnosis.

The determination of the fetus genetic condition with respect to sex and/or potential genetic diseases caused by known or unknown small mutations or chromosomal abnormalities requires the examination of the fetal chromosomes isolated from fetal cells. Chromosomal examination is generally performed using cytogenetic technologies, which require the sampling of living cells of the fetus followed by cell culturing for some weeks in order to grow a sufficient amount of cells for further analysis. The detection of small mutations however can be performed with a small quantity of cell sample using polymerase-chain-reaction (PCR) technologies combined with PCR-sequencing or other post-PCR mutational detection techniques. The sampling of living fetal cells is usually performed during the second trimesters of pregnancy using standardised surgical procedures in specialised outpatient clinics. A small biopsy is taken either from tissue of the placental chorionic villi (chorionic villi sampling, CVS) or from the amniotic fluid (amniocentesis) by inserting a needle through the mothers abdominal wall. A third method involves the punction of the umbilical to obtain fetal cells from the cord blood after the 18 th week of gestation.

Unfortunately, spontaneous miscarriages result with a frequency of about 0.5 to 1.5% as a matter of this invasive procedure. In rare cases other serious conditions have occurred harming the mother's and the fetus' health.

Besides these invasive sampling procedures non-invasive procedures of risk assessment for fetal chromosomal abnormalities are common, e.g. the biochemical screening for alpha-fetoprotein, human chorionic gonadothropin and unconjugated estriol in the maternal blood, known as the "triple-test". Other methods include the analysis of fetal cells including the recovery of nucleated fetal cells from mothers blood stream. Extracted from the mother's blood these cells can serve as a source of information about the genetic status of the developing fetus and therefore can serve for prenatal diagnosis.

A variety of cell types of fetal origin cross the placenta during pregnancy and circulate within the maternal peripheral blood (Bianchi, D, W. and Klinger, K.W. in: Genetic Disorder and the fetus: Prevention and Treatment, 3^{rd} edition, Baltimore/ London: The John Hopkins University Press 1992; B. Tutwschek, Nichtinvasive Pränataldiagnostik an fetalen Zellen im mütterlichen Blut, Gynäkologie (1995) 28: 289-301).

Usually, the target cells for prenatal diagnostics are trophoblasts, nucleated fetal red blood cells, granulocytes and/or a subpopulation of white blood cells, i.e. polymorphonuclear leukocytes: neutrophils, basophiles and eosinophiles (An investigation of methods for enriching trophoblast from maternal blood, Prenatal Diagnosis, Vol. 15: 921-931 (1995); German patent DE 4222573; international patent application WO 96/27420; US-Patent 5,714,325).

Another, but less common method to obtain fetal cells, is the collection of transcervical cells (Non or minimally invasive prenatal Diagnostic on maternal blood samples or transcervical cells, Prenatal Diagnosis, Vol. 15: 889-896 (1995)).

The general usability of these cell types is greatly hindered due to their limited concentration in maternal blood, which is estimated at between 1 in 10⁵ and 1 in 10⁸ circulating cells. A more precise estimation by Hamada et al. (Fetal nucleated cells in maternal peripheral blood: frequency and relationship to gestational age. Human Genet. 1993 June: 91(5) 427-32) gives a number of nucleated fetal cells at 60-600 per ml of maternal blood or 1200-12000 in a typical 20 ml sample. Several reports could confirm these figures but never have been able to collect such a number of fetal cells with high recovery rates. Several groups however reported on an increased rate of feto-maternal cell traffic in aneuploidy conditions. This suggests fetal cell separation to be more successful in the presence of such abnormalities than under normal conditions.

In order to conduct a genetic analysis, low fetal cell numbers necessitate removal of maternal cells respectively enrichment of fetal cells. Since most of the fetal cell types cannot be distinguished from maternal cells on the basis of morphology alone, methods such as density gradient centrifugation or charge flow separation (DE-OS 4222573; WO 96/27420, WO 96/12945) have been applied in order to enrich fetal cells. The charge flow separation exploits differing surface charge densities of interesting cells which are sorted directly into distinct tubes for further analysis. Nevertheless, as a draw back this technique implies the risk of as well as false positive as false negative results.

More specific methods are based on incubating the blood sample with a target cell specific, fluorescent labelled antibody, which enables the selection of the target cell by a fluorescent activated cell sorting (FACS). This system is based on the detection of fluorescence of each single cell passing an adequate detection system. According to the fluorescence the cells are electrically charged and selected into different portions. Another method utilises metallic beads labelled, target cell specific antibodies, which are identified with magnetic activated cell sorting (MACS). These techniques are known e.g. from the international patent application WO 9107660 or German patent application 42 225 73.

However, FACS and MACS frequently dependent on the availability of monoclonal antibodies and require considerable expertise. The antibody fraction can exhibit a non-specific binding to other cellular components which leads to a high signal and noise ratio and impairs the later detection. Furthermore, antibodies binding with fetal cell surface antigen, e.g. γ-chain of fetal haemoglobin, often also bind to maternal cells. Thus, samples obtained by commonly known cell sorting contain contaminating maternal cells.

Examples of methods using one or a combination of several above mentioned principals are described, e.g. in Yeoh et al., Prenatal Diagnosis 11:117-123 (1991); Mueller et al., Lancet 336:197-200 (1990) for the use of murine monoclonal antibodies for the isolation of fetal trophoblasts. Price et al., American Journal of Obstetrics and Gynecology 165:1731-37 (1991) outline flow sorting of fetal nucleated erythrocytes on the basis of cell size and granularity, and transferin receptor and glycophorin A cell surface molecules. PCT publication WO 91/07660 describes a method for isolating fetal nucleated erythrocytes using an antigen present on the cell surface of fetal erythrocytes. The PCT publication WO 91/16452 and US-patent 5,153,117 disclose a method using a combination of different antibodies to fetal cells which were labelled with different fluorochromes.

The US-patent 5,858,649 describes a method of enriching fetal cells from maternal blood and identifying such cells. It comprises the sampling of maternal blood and the amplification of a target specific, i.e. fetal cell specific, ribonucleic acid. Later in-situ hybridisation of the cells take place with a hybridisation medium containing labelled ribonucleic acid probes complementary to the target ribonucleic acid of fetal cells. Nevertheless, since non-hybrised labelled ribonucleic acid probes remain within the cell they lead to a high signal/noise ration and thus to a low reliability of analysis results.

This method suggests a step of enrichment of fetal cells either by positive or by negative selection preceding the hybridisation i.e. density gradient centrifugation or flow cytometry. However, preferably, fetal cells are separated from maternal cells by FACS or MACS technology using antibodies against fetal antigen or antigens, being enriched on fetal cell surface. The techniques for cell separation suggested by US 5,858,649 exhibit the drawbacks and problems outlined above.

Sokol et al. (Deborah L. Sokol, Zangh X., Ponzy, L, Gewirtz A.M.; Real time detection of DNA•RNA hybridisation in living cell; Proceedings of National Academy of Science 1998, 95 pp. 11538-11543) provide a method to detect hybridisation of a labelled nucleic probes with target nucleic acid. As labelled sequences they utilise so called molecular beacons which are subsequently detected by spectrofluorimeter and or confocal laser scanning microscopy. These detection systems are time consuming and thus are not suitable for the routine practice of analysing high quantities of samples. Furthermore, they do not allow for a separation of target cell from non target cell.

Therefore, a primary objective of the present invention is to provide a method which improves the identification and/or separation of cells and to improve genetic diagnosis, especially direct genetic diagnosis in living cells. A further objective of the invention is to improve the diagnosis by providing a device and a method for a better sampling and/or processing of the investigated cells.

The underlying problem is solved by providing a method for cell identification according to claim one.

The basic idea of the invention is to identify target cells and to detect genetic alterations within target cells in vivo by hybridising target sequences with complementary or partly complementary labelled sequences and subsequently detect the hybridised labelled sequence within the cell by flow cytometry. A step of cell sorting according to the detection of the hybridised molecular beacon and further analysis may follow.

It is a special advantage of the invention to improve the identification of fetal cells within a maternal blood sample and therefore to enable the distinction between maternal and fetal cells, especially by overcoming the drawbacks of insufficient specificity and high signal/noise ratio. This method identification with subsequent cell separation is less time consuming and requires less expertise than common practices.

In a further advantage the presented method enables to increase the overall sampling rate of target cell within a blood sample per patient.

Another advantage is the simultaneous detection of different cell types or lines in a fast multiplex assay which can be combined with the detection of various genetic differences within these cells at once.

Further to the detection a quantification of the hybridisation signal derived from hybridised labelled sequences can be performed, giving information about the expression rates of target sequences. Therefore, the quantification can also provide further discrimination criterion between target and non target cells. Even rare sequences can be detected.

Thus, in a preferred embodiment the invention allows for the diagnosis of diseases which are due to or characterised in the transcription of genes which are not expressed in the wild type cell, or diseases characterised in the absence or altered expression or expression rates of certain genes or parts by quantifying the level of abundance of the transcript products.

Therefore, the method according to the invention can provide for prenatal diagnosis.

Subsequently to the detection and or quantification the separation of the target sequence carrying cell may be advantageous, e.g. to isolate fetus derived cells out of a maternal blood sample. The cell separation can be conducted e.g. by photographic or visual methods. In a preferred embodiment the cell sorting is performed with commonly known fluorescence activated cell sorting (FACS; e.g. international patent application WO 9 107 660 or German patent application 42 22 573).

Generally, nucleic acid hybridisation techniques are based on the ability of single-stranded nucleic acid to pair with a complementary nucleic acid strand. Therefore, a hybridisation reaction prescribes the development of labelled complementary specific sequences (subsequently also labelled sequence) directed against a target sequence or combination of complementary specific sequences in order to identify the presence or absence of target sequences of genes (DNA) and/or their transcribed polynucleotide sequences (RNA) or even the presence of a specific mutation within a sequence and/or a transcribed gene product. The target sequence can be a wild type are genetically altered nucleic acid.

According to the invention a mixture of at least two complementary sequences directed each against a specific target sequence (wild type or mutation) can be used which are labelled each with a different fluorochrome. These fluorochromes advantageously exhibit a different fluorescent emission. This can allow for the detection of different target sequences in one assay. Therefore, also the different target sequences carrying cells can be identified and/or sorted in one assay (multiplex assay).

In a preferred embodiment a selected labelled sequence can be complementary to a genetic marker of the target cell, for example for the gender or any fetal specific gene transcript or for other genetic characteristics of the target cell. Also labelled sequences can be directed against bacterial or viral nucleic acids being included in the target cell or part thereof. Examples for viral targets can be the human immunodeficiency virus (HIV), or the herpes or hepatitis virus. Other detection targets of particular interest and therefore included are polynucleotide transcripts of the X- or Y-chromosome or the chromosome 1, 13, 16, 18 and 21.

In a preferred embodiment the target nucleotides are hybridised with a complementary labelled sequence that has a nucleic acid target complement sequence flanked by members of an affinity pair or arms, that under assay conditions - in the absence of the target sequence - interact with one another to form a stem duplex. Hybridising of the sequence with the target sequence produces a conformational change in the sequence forcing the arms apart and eliminating the stem duplex. Due to the elimination of the stem duplex the sequence becomes detectable.

These detectably labelled dual conformation oligonucleotide sequences are disclosed in US patent 5,925,517, which is hereby fully incorporated in the text in terms of disclosure.

In a preferred embodiment of the invention the above mentioned oligonucleotide (which subsequently will be referred to as molecular beacon (MB)) matches a donor and acceptor chromophores on their 5' and 3' ends. In the absence of a complementary nucleic acid strand, the MB remains in a stem-loop, i.e. stem-duplex, conformation where fluorescence resonance energy transfer prevents signal emission. On hybridisation with a complementary sequence, the stem-loop structures opens increasing the distance between the donor and the acceptor moieties thereby reducing fluorescence resonance energy transfer and allowing detectable signal to be emitted when the MB is excited by light of appropriate wavelength. Without hybridisation the donor and acceptor remain in distance and due to the above described effect of quenching no fluorescence signal is detectable. Thus, non-hybridised MB do not emit fluorescent light.

Due to this effect a washing or otherwise removing of non-hybridised labelled oligonucleotides can be avoided. Even a non specific binding of hybridising medium does not result in a relevant background signal otherwise impairing the signal to noise ratio during subsequent cell separation.

It is an advantage that the method according to the invention does not require the availability of monoclonal antibodies. Nevertheless, substituting labelled antibodies with labelled probes the cell separation protocols commonly known for FACS or MACS can be applied.

In a special embodiment of the invention fetal cells are separated from a variety of sample specimens including maternal peripheral blood, placental tissue, chorionic villi, amniotic fluid and embryonic tissue. A preferred specimen is a maternal peripheral blood sample. The invention can be used to identify and sort fetal nucleated red blood cells, but any other fetal cell type carrying a nucleus and having gene transcription activity can be included with no further difficulties.

In order to distinguish a fetal cell at least a suitable target sequence can be chosen within the cell, which is either specific in quality and/or quantity to fetal or embryonic cells. The preferred target sequences to detect are fetal-gene-specific chromosomal transcripts like messenger ribonucleic acids (mRNAs) or ribosomal ribonucleic acids (rRNAs), without limiting the invention to them, e.g. the mRNA for fetal hemoglobin (HbF) or embryonic hemoglobin. In general, different expression levels of genes can be utilised which are specifically active in the fetal cell.

In one embodiment of the invention labelled sequences complementary to cell line or cell type marker can be used in one assay together with labelled sequence complementary to genetically altered target sequences. Thus, a maximum information can be obtained at once: i.) The presence or absence of a target cell type or line and ii) The absence or presence of a genetically altered target sequence. Therefore, a cell identification and a direct genetic diagnosis can be performed in a combined assay.

In this combined assay two sets of molecular beacons can be used, e.g. one set can be directed against target cell lines specific RNA, e.g. against fetal hemoglobin mRNA, and a second set is directed against a DNA sequence of interest, e.g. a sequence with single nucleotide alteration. Both sets are labelled with differently coloured fluorophores. The cell identification and possible subsequent cell separation can be performed according to a FACS protocol. It allows the identification of certain genetic abnormalities as a direct genetic diagnosis of the gene DNA and the analysis of the expression level of the genes by quantifying the mRNA simultaneously. Thus, genetic abnormalities can be detected in an 'online' fashion during the FACS procedure. In case of fetal cells it allows to directly determine a certain genetic condition of the fetus without a genetic testing procedure following the separation from maternal cells.

In a further embodiment of the invention multiple target sequences can be selected detecting different cell types, which for example share the same origin. This for example allows for multiple fetal cell types to be collected at once out of a maternal blood sample, thereby maximising the total amount of cells being collected compared to a technique that is optimised for collecting specific fetal derived cells like nucleated red blood cells (NRBCs).

In another embodiment of the invention, the blood samples for later cell identification and genetic analysis are taken in a test tube comprising at least one compartment with incubation media containing hybridisation media including at least one group of labelled sequences, preferably molecular beacons.

This test tube provides the advantage of shortening the period between sampling and hybridisation considerably, which can result in an increase in reliability and quality of the test results.

Advantageously, the test tube comprises two or more compartments each with different incubation- or test-solutions, e.g. fixation, or staining solution and/or anticoaglutants. While primarily, the samples is incubated with one media, after a defined period if time, a second media from a second compartment can have access to the sample e.g. by opening a membrane separating the compartments.

Prior to cell identification and/or genetic analysis it can be advantageous to conduct a quantitative pre-test to estimate the proximate concentration of target cells to be expected in the cell sample. In case of fetal analysis it can be also advantageous to determine the sex of the fetal cells prior cell identification in order to select suitable molecular beacons for further steps of cell identification and genetic analysis.

In a preferred embodiment this pre-test is conducted as a quantitative online-PCR approach determining the Ct value of the PCR for fetal hemoglobin which is further on compared to a positive and negative blind sample. The sex of the fetus can be preferably determined by using molecular beacons complementary to the middle of PCR amplicons from mRNA sequences of zfy.

In one embodiment the method according to claim one the invention is combined with current methods of negative separation such as density gradient centrifugation and/or techniques like antibody derived magnetic separation (MACS) of unwanted cells and with positive separation techniques on the basis of physical parameters like the cell surface charge by using a free buffer-flow electrophoresis device. Advantageously the step of negative selection of maternal cells is conducted before the in situ hybridising and prior to fluorescence activated cell-sorting procedure (FACS).

Methods of negative selection of unwanted cells include the application of a hypotonic shock, which leads specifically to the lysis of erythrocytes first. Lysis solutions are readily commercially available e.g. from PARTEC, DAKO, Caltaq or MEDAC.

Another method to remove enucleated red blood cells is the complement system. Complement, a group of serum factors that can destroy antibody marked cells, is used strictly for negative selection, i.e. elimination of unwanted cells.

Alternatively or additionally specific cells, here monocytes can be reduced by the LME Treatment. LME (L-Leucin-methyl-ester) is a lysomotropic agent and destroys monocytes.

Due to the low absolute number of rare cells within a group of cells of the main population it can be of advantage to pre-enrich the rare cells prior FACS or other sorting procedures. This positive selection can be performed using for example intracellular staining techniques (US 5,422,277) or magnetic cell sorting (MACS).

With MACS, sell sorting from complex cell mixtures, such as peripheral blood, hematopoietic tissue or cultured cells is possible. Since small magnetic particles (20 - 150 nm in diameter) exhibit faster kinetics of the cell-bead reaction, a lower degree of non specific cell bead interactions, a lower risk of non specific entrapping of cells in particle aggregates, and less adverse effects of particles on viability and optical properties of labelled cells when compared with large magnetic particles (0,5 - 5 µm in diameter), they can be applied advantageously. Especially a MACS technology with small super- paramagnetic particles and high gradient magnetic fields is advantageous. Nevertheless, it can be combined with large magnetic particles as well for example large magnetic beads from Dynal and depletion columns from Miltenyi.

Furthermore, MACS can be used for negative selection, i.e. for example for the depletion of white blood cells. Since CD45 antigen is expressed on all cells of hematopoietic origin except erythrocytes, platelets and their precursor cells, CD45 Micro Beads can be used for the depletion of leukocytes from peripherel blood. To improve the efficient depletion of all leucocytes a combination of CD45 and CD15 Micro Beads is recommended due to the weak expression of CD45 in the granulocyte / monocyte lineage. Therefore, the combination of MicroBeads can result in an enrichment of fetal erythroblasts from maternal blood.

Prior to MACS it is advantageous to supply the relevant buffer with a resuspending medium, such as EDTA, bovine serum albumin (BSA) or serum, to achieve a sufficient resuspension of the cells. Furthermore it is recommended to remove dead cells.

In order to achieve a pre-selection of the cells prior to FACS, also charge flow separation technique, especially in the continuous free-flow electrophoreses method as disclosed US patent 4,061,560, (fully incorporated into the text hereby) can be used. It can be either used without or including staining with target cell specific antibodies (ASEC). The latter is reviewed by Hansen et al., 1982, (Antigen-specific electrophoretic cell separation (ASECS): Isolation of human T and B lymphocyte subpoulation by free-flow electrophoresis after reaction with antibodies. J. Immunol. Methods 51 : 197 - 208 ). The method can be improved by using a second antibody directed against the first in a so called "sandwich technique". If this is still not sufficient, a third antibody can be used, directed against the second.

Alternatively specific antibodies with side groups containing a higher negative charge than a normal antibody can be used. Furthermore, antibodies coupled with very small magnetic particles can be advantageous.

For the final separation step of cells flow cytometry is used. Preferably, fluorescence activated cell sorting is performed. However, fluorescence is just one possible staining system since other dye systems may also be employed (US-patent 4,933,293) and no staining is necessary for light-scatter measurements or electrical sizing.

Flow cytometry apparatus are commercially available e.g. from MICROCYTE, Becton Dickinson's FACScan, FACStrak, FACSort, FACSCalibur, FACStar, FACSVantage, Bio-Rad's BRYTE-HS, Coulter's PROFILE and EPICS, Cytomation's MoFlow, Ortho's CYTORON and Partec's PAS machines. The PAS- System from PARTEC (Germany) is preferred.

It may be advantageous to perform the flow cytometry with target cells being stained with antibodies additionally to the labelling of target sequences with molecular beacons. This might include fixation of cells prior staining. The antibodies can be directed against intracellular substances or surface markers. Treatments of cells with a fixative as described in US Patent NR. 5422277 and 6004762 allow antibodies or other desired components to enter the cell through the cellular membrane.

Direct staining with flourochrome -conjugated antibodies against specific cellular determinants is preferable. The availability of many different dyes suitable as labels for immunofluorescence enables the simultaneous measurement of many subpopulations in one sample. Reviews about dyes for immunofluorescence can be found in Glazer et al. (Fluorescent tandem phycobiliprotein conjugates. Emission wavelengths shifting by energy transfer. Biophys J. 1983 Sep.. 43 (3). P 383-386), Recktenwald et al. (Peridinin chlorophyll complex As fluorecent label. US Patent No. 4876190. October 24,1989), Recktenwald et al. (Biological pigments As fluorescent labels for cytometrie. New Technologies in Cytometrie and Molecular Biology, Gary C. Salzman, Editor , Proc. SPIE 1206 P 106-111, 1990), Waggoner et al. (A new fluorescent antibody label for three - Color flow cytometrie with a single laser. Ann N Y Acad Sci 1993 677 P185-193), Haugland et al. (Spectra of fluorescent dyes used in flow cytometry. Methods Cell Biol 1994. 42 Pt B P 641- 663) and Roederer et al. (10-parameter flow cytometry to elucidate complex leukocyte heterogeneity. Cytometrie 1997. 29 (4) P 328-339.)

It is also possible to combine immunofluorescence with DNA staining such as Propidiumjodid and DAPI are fluorescence dyes who stain DNA. See also Rabinovitch et al. (Simultaneous cell cycle analysis and two color surface immunofluorescence using 7-amino-actinomycin D and single laser.J Immunol. 1986 Apr. 15 136 (8). P 2769-2775).

In order to allow staining antibodies against intracellular compounds to penetrate the cell membrane cells have to be fixed and the membranes have to be permeabilized. For several application fixation and permeabilisation of cell membranes saponin can be used successfully, including assessment of cytokines (see also Willingham: An atlas of immunofluorescence in cultured cells. Vol. II Academic press). As a cross linking fixative formaldehyde can be applied with allows for good preservation of cell morphology. Alternatively other detergents, like NP40 in combination with fixation by formaldehyde, or of organic solvents, like 70 % methanol or ethanol/acetic acid (95/5), which fix and permeabilise cells in one step can be used.

Especially for staining RNA and DNA, fixation with alcohol is preferable. Sometimes even a combination of different fixation/permeabiiisation steps might be useful, e.g. 70 % alcohol followed by formaldehyde / Tween 20 for BrdU-staining.

In order to overcome problems with background staining absorption of polyclonal antibodies on liver powder (acetone precipitate of liver) or on irrelevant cells (2:1, volume of antibody solution (1mg/ml) : packed cells) can reduce unspecific staining. Optimally specific staining can be blocked by preincubation of the antibodies with molar excess of purified antigen.

### Definitions:

The term nucleic acid refers to sequences of nucleotides of all kind and thus comprises oligomers and polymers of desoxyribonucleotides, as well as all kinds of ribonucleotides. Also nucleotides with analoga, chromosomes and viral or bacterial nucleic acids or parts thereof, plasmids, recombinant nucleotides and all kinds of synthetic sequences are included.

The term target cells refers to cells of interest, which are to be selected or purified respectively. In case of fetal cells as target cells they include especially trophoblasts, nucleated fetal red blood cells, granulocytes and/or a subpopulation of white blood cells, i.e. polymorphonuclear leukocytes such as neutrophils, basophiles and eosinophiles.

The term target sequence refers to all nucleic acids, which are to be hybridised with the labelled complementary sequence. This term comprises sequences specific for the target cells in terms of quality and/or quantity. It comprises wild type sequences as well as genetically altered nucleic acids of all kind.

The term labelled sequence refers to nucleic acids complementary or partially complementary to target sequences, which are labelled with at least one marker such as chromophores, fluorophore, magnetic particle or others allowing the later detection.

The term molecular beacon refers to a labelled sequences according to one of the claims of US patent 5,925,517.

The term RNA refers to all kinds of RNA, including mRNA and rRNA as well as derivates and parts thereof..

The term DNA refers to all kinds of naturally occurring or synthetically derived DANN as well as derivates and parts thereof.

The term hybridisation refers to the phenomenon that single strand nucleic acids or parts thereof are forming pairs with complementary or partly complementary single nucleic acid strands. In situ hybridisation refers to hybridisation under conditions maintaining the cell substantially intact.

The term fluorescence refer to emission of detectable radiation as a result of excitement with radiation of a different wavelength than the emitted.

### Examples

### Example 1.

Detection of chromosomal aberration using molecular beacons in flow cytometrie (flow chart I).

In case of positive zfy results obtained in the pre-test described under step 2) a zfy specific molecular beacon (FITC) is synthesized (see under point 3)).

A negative result in the pre-test indicates a female and therefore, a HbF mRNA (FITC) specific molecular beacon is chosen.

After the delivery of molecular beacons into the enriched fetal cells, described under step 6) a HbF specific antibody (PE) and a nuclear staining with DAPI (PARTEC Germany) improves the determination of the fetal cells (see under step 7).

The target cells are identified, gated and automatically separated according to the protocol of the operating manual for the PAS-III, (PAS-III: Particle Analysing and separation System, Operating Manual; Partec Germany, see also under step 8).

Finally a diagnosis is conducted by FISH analysis, described under step 9) by using commercially available region-specific large-insert clones (Vythis, USA) for the detection of trisomy 21.

### Example 2

Direct mutation analyses using cystic fibrose gene specific molecular beacons in flow cytometrie (see flow chart II)

Pre-enrichment of the fetal cells was performed as described under step 1.)

As described in flow chart II one molecular beacon is used to distinguish fetal from maternal cells. In case of a positive result in step 2.) a zfy specific mRNA molecular beacon (FITC) is used to differentiate between fetal and maternal cells. As a second parameter for distinguishing fetal cells from maternal cells a fetal specific mRNA - molecular beacon for the HBF-gene and a DAPI nuclear staining is applied.

The molecular beacon for the determination of the wild type sequence is labelled with EDANS and a mutation specific beacon is labelled with the fluorescens HEX (according to step 3).

A fetal cell is to be distinguished by FITC and DAPI fluorescence. The genetic status is determined by the subsequent possible combinations: 1 DAPI and FITC positiv (fetal cell) 1a: HEX positiv, EDANS negativ (fetal cell wildtyp homozygous). 1b: HEX positiv, EDANS positiv (fetal cell, wildtyp and mutation) 1c: HEX negativ and EDANS positiv (fetal cell, mutation homozygous).

### Detailed description of procedural steps

### 1. Blood sample

Approximately 30 ml blood from a pregnant woman after 7 to 15 week of gestation is collected in a vacuum - test - tube containing one or more anticoaglutants e.g. acid-citrate-dextrose (ACD), ethylendiamine-tetracid (EDTA), heparin and/or citrate-phosphate-dextrose-adenine (CPDA-S-Monovette, Sarsted, Germany). The blood probe is processed and analysed within 48 hours of sampling.

### 2. Quantifying fetal mRNA in maternal blood (pre-test)

300 µl of the whole blood sample is added to a 1.5 ml microfuge tube containing 900 µl RBC Lysis solution (Quantum Prep ApuaPure RNA Isolation, BioRad) and mixed by inverting the tube for 10 min at room temperature. After centrifugation at 13,000-16,000 x g in a microcentrifuge for 20 sec the supernatant is removed with a pipet leaving behind the visible white cell pellet and about 10-20 µl of residual liquid. The white cells are re-suspended by vortexing vigorously until the pellet has disappeared.

300 µl RNA lysis solution (Quantum Prep ApuaPure RNA Isolation, BioRad) is added to this mixture by pipeting up and down for 3 times. 100 µl of DNA-precipitation solution (Quantum Prep ApuaPure RNA Isolation, BioRad) is added to the cell lysate, mixed by inverting the tube and placed into an ice bath for 5 min before centrifugation at 16,000 x g. The precipitated protein and DNA form a tight pellet. The supernatant is placed into a clean sterile 1,5 ml micorcentrifuge tube containing 300 µl pure isopropanol. The sample is mixed by inverting 30 times and centrifuged for 3 min at 16,000 x g. Total RNA is visible as a small translucent pellet. The supernatant is poured and the tube is dried in an absorbent paper. The pellet is washed twice with 70% ethanol, air dried for 30 min and stored at -80°C until used.

First strand complementary DNA is synthesized by priming with random hexamers (Clontech). The air dried and frozen RNA sample is resuspended in an 8.5 µl solution consisting of :
1 µl 50 µmol/l random hexamers
0.2 µl 0.1 mol/l dithiothreitol (DTT)
0.05 µl Rnase inhibitor (Rnasin, Promega) and
7.25 µl sterile nuclease free water.

The hexamers are annealed by incubating the sample at 70°C for 5 min and quenched on ice. Reverse transcription is performed by addition of 11.5 µl containing
4 µl 25 mmol/l MgCl₂,
2 µl 10x PCR buffer II (Perkin Elmer),
4 µl 2 mmol/l dNTPs (Amersham Pharmacia),
1 µl RNase inhibitor (Promega) and
0.5 µl Maloney murine leukaemia virus (MMLV) reverse transcriptase (Gibco BRL)
and incubated at 37°C for one hour. The reaction is stopped by heating to 95°C for 5 min.

Prior to be used in a RT-PCR, 5 µl RNA Hydration Solution (Quantum Prep ApuaPure RNA Isolation, BioRad) is added to the RNA. The mixture is subsequently vortexed heavily and 5 µl are used in a 50 µl PCR experiment.

A multiplexed quantitative real-time PCR is performed utilizing molecular beacons that are complementary to the middle of PCR amplified fragments from mRNA sequences of beta-actin, zfy and HbF. The length of the arm sequences of the molecular beacons is chosen in order to allow a stem being formed at the annealing temperature of the PCR (table 1) whereas the length and sequence of the loop is chosen to provide a probe-target hybrid being stable at this step of PCR. The design of molecular beacons has been tested before by thermal denaturation profiles using loop-antisense oligonucleotides and a real-time thermal cycler (BioRad). Only molecular beacons showing desired thermal profiles are included in the subsequent PCR at concentrations similar to the amplification primers

During the denaturation step of the PCR, the molecular beacons assume a random coil conformation and fluoresce. As the temperature is lowered to allow annealing of the molecular beacon to the target sequence at the single stranded PCR fragments, loop-target hybrids are formed which are able to continue to fluorescing. Superfluous molecular beacon however rapidly form stable intra-molecular stem-hybrids that prevents them from fluoresce. As the temperature raises to allow primer prolongation, the molecular beacons dissociate from the target sequences and do not interfere with the polymerization step. A new molecular beacon hybridization step takes place in every annealing step during PCR cycling while the increase of the resulting fluorescence is monitored and indicated the amount of the accumulated target amplicon.

In a multiplexing PCR experiment, at least two different target sequences are simultaneously amplified in one tube. At least two different molecular beacons are used simultaneously, each of them labelled with a different fluorescent dye with no spectral overlap at emission wavelength. The resulting fluorescence is monitored at the appropriate wavelength of each of the molecular beacons during the annealing step of the PCR.

Multiplexing of HbF and zfy PCR is combined with a beta-actin PCR as an internal control PCR. Beta-actin is widely used its mRNA is ubiquitously abundant in almost every cell type. Beta-actin amplification therefore not only indicates a successful polymerase chain reaction but is highly proportional to the total amount of target cells used in the PCR.

The primers and molecular beacons used in the real-time quantitative PCR experiment are shown in table 1. In one q-RT-PCR experiment the amount of zfy-gen-specific mRNA is quantified and the result is compared to a positive and a negative control sample, which is the total mRNA from a blood sample of a mother carrying a male or a female fetus respectively.

Real-time quantitative RT-PCR is performed by using the iCycler Thermal Cycler (BioRad). The PCR for zfy mRNA and HbF mRNA is conducted in separate reactions but multiplexed with a PCR for beta-actin. Each 50 µl reaction contained
5 µl of the relevant template cDNA,
1.0 µM of primers for amplifying either zfy or HbF,
0.25 µM of the primers for amplifying beta-actin,
0.2 µM of either the zfy-molecular beacon or 0.5 µM of the HbF-molecular beacon,
0,2 µM of the beta-actin molecular beacon,
0.25 mM of each dNTPs,
2.5 units of AmpliTaq Gold (Perkin-Elmer),
4 mM MgCl₂,
50 mM KCI and 10 mM Tris-HCI at pH 8.3.

The 50 µl volume reaction is loaded into the appropriate PCR-tubes and placed into the iCycler (BioRad). The cDNA is denatured and heating to 96°C for 10 min activates the Taq Polymerase. In a further 50 cycle PCR, denaturation is performed at 95°C for 30 sec, annealing of the primers and molecular beacons at 60-68°C for 30 sec and extension at 72°C for 30 sec. Fluorescence data is acquired during the annealing steps of the reaction and the level of fluorescence is monitored as a function of cycle number.

The reaction without any template does not exhibit any increase in fluorescence and functions as a baseline. Over a wide range of template concentrations the cycle with a fluorescence signal exceeding detectably over the baseline is inversely proportional to the logarithm of the initial number of template molecules. The level of beta-actin fluorescence in each well is a suitable further control parameter indicating the amount of total cDNA target used for the PCR.

In order to compare the fluorescent signals resulting from the synthesis of each target amplicon, the fluorescence intensity (F) of each molecular beacons is normalized by calculating (F - Fₘᵢₙ)/(Fₘₐₓ - Fₘᵢₙ). Thus, the value "0" represents fluorescence before target amplification, and "1" represents the maximum level of fluorescence after PCR. For each molecular beacon the threshold cycle is determined when the intensity of the fluorescent signal exceeds 10 times the standard deviation of the background baseline fluorescence. For quantification of the target a comparison of the determined threshold cycles with a standard curve is performed.

### 3. Preparation of molecular beacons

25- to 30-nucleotide-oligodesoxynucleotides (see table 1) are synthesized consisting of a 15- to 20-nucleotide-target sequence (antisense to mRNA) sandwiched by a complementary 5-nucleotide-arm sequence (stem sequence), being covalently linked to a fluorescent dye (fluorescein or EDANS) at the 5'-end and to a DABCYL as a quencher dye at the 3'-end. This protocol results in a molecular beacon of the following general formula: fluorescein-5'-GCGAGC-target-sequence-GCTCGC-3'-DABCYL.

The initial oligonucleotide for this synthesis of the molecular beacon is conducted contains a sulfhydryl group at its 5'-end and a primary amino group at its 3'-end. Its synthesis is conducted with a standard A-, C-, G-, T-phosphoamidate chemistry on an automatic DNA synthesizer (Perkin Elmer 394) using 1-dimethoxytrityloxy-3-fluorenylmethoxycarbonylaminohexane-2-methylsuccinyl-long chain alkylamino-controlled pore glass (C7-CPG, Perseptive Biosystems) as the 3'-aminomodifier. A trityl-hexylthiol linker ((S-trityl-6-mercaptohexyl)-(2-cyanoethyl)-(N,N-diisopropyl)-phosphoamidate, PerSeptive Biosystems) is coupled as a final step to the nucleotide' s 5'-end.

Subsequently, the oligonucleotide is detached from the support using 28% ammonium at 55°C for 6 h. This treatment also removes the protective moieties at the amino and phosphate groups except for the trityl moiety at the 5'-sulfhydryl end. The detached oligonucleotides are purified by reverse phase cartridge column (Waters Sep-Pak C18, Millipore) and then fractioned by HPLC on a C18 column with a linear gradient of 5-40% acetonitrile dissolved in 0.05 M triethylammonium acetate (pH 7.0) running for 30 min at a flow rate of 1.0 ml/min at 40°C and detected at 254 nm.

About 200 nmoles of the dried oligonucleotide was dissolved in 0.5 ml of 0.1 M sodium bicarbonate, pH 8.5. Subsequently the mixture is incubated with 20 mg of DABCYL (4-(4'-dimethylaminophenylazo)benzoic acid) succinimidyl ester, (**Molecular Probes**) in 0.1 ml N,N-dimethylformamide in 0.01 ml aliquots at 20 min intervals. The mixture was kept for 3 days at room temperature in the dark. Excess DABCYL is removed from this mixture by passing through a Sephadex G-25 column (Nap-5, Amersham-Pharmacia), equilibrated with 0.1 M triethylammonium acetate (pH 6.5). The eluate of approximately is filtered through a 0.2 µm filter (Centrex MF-0.4, Schleicher & Schüll) and loaded on a C-18 reverse phase HPLC column (Waters), utilizing a linear elution gradient of 20% to 70% 0.1M triethylammonium acetate in 75% acetonitrile (pH 6.5) in 0.1 M triethylammonium acetate (pH6.5) for 25 min at a flow rate of 1 mll/min. The absorption by DABCYL is monitored by spectrophotometry. The peak absorbing at 260 and 491 nm is collected.

In order to remove the trityl moiety from the sulfhydryl group at the 5'-end, the dried and DABCYL-coupled oligonucleotides are dissolved in 0.25 ml 0.1 M triethylammonium acetate (pH 6.5) and incubated with 0.01 ml of 0.15 M silver nitrate for 30 min at room temperature. To this solution 0.015 ml of 0.15 M DTT is added. The supernatant is removed from the pellet by spinning and transferred into a solution consisting of 40 mg 5-iodoactamidofluorescein (Molecular Probes) in 0.25 ml of 0.1 M sodium bicarbonate (pH 9.0). Incubation is performed at room temperature for one day in the dark.

Excess fluorescein is removed by gel exclusion chromatography (Nap-5, Amersham Pharmacia) and the oligonucleotide are purified by HPLC. The fraction absorbing at wavelength 260 nm and 491 nm is collected. It is precipitated and re-dissolved in 0.1 ml TE buffer. The yield is estimated by determining the absorbance at 260 nm.

### 4. Density Gradient Centrifugation

15 ml of peripheral maternal blood is layered carefully in a 50 ml conical centrifuge tube containing 15.0 ml Histopaque ® - 1119 (Sigma Diagnostics) at room temperature which is centrifuged at 500 x g for exactly 30 minutes at room temperature. Centrifugation at lower temperatures, such as 4°, may result in cell clumping and poor recovery.

After centrifugation the upper layer to within 0,5 cm of the opaque interface containing mononuclear cells is aspirated. The white band, including nucleated erythroid cells and lymphocytes, is directly below the plasma layer.

Histopaque®- 119 containing a broad diffuse band containing other nucleated erythroid cells and immature red cells with densities heavier than the white layer but lighter than the mature erythrocyte pellet, which is immediately below the nucleated cells and the erythrocyte pellet at the very bottom of the tube.

The cells are transferred in a new tube add 30 ml Phosphate Buffered Saline solution with 0,1 % BSA is added, mixed by gentle aspiration and subsequently centrifuged at 250 x g for 10 minutes. The supernatant is discarded.

The pellet is resuspended with 10 ml Phosphate Buffered Saline solution with 0,1 % BSA and centrifuged at 250 x g for 10 minutes again. The step of washing is repeated and the supernatant is discardes. The cell pellet is re-suspended in 750 µl Buffered Saline solution with 0,1 % BSA.

### 5. Magnetic activated cell sorting (Depletion, Separation)

### Depletion

In order to improve the efficient depletion of all leucocytes a combination of CD 45 and CD15 is used (Miltenyi Biotec GmbH, Bergisch Gladbach, Germany; Order No. 458-01 and No. 466-01).

To reduce the forming of aggregates a resuspension buffer is supplemented with EDTA and bovine serum albumin (BSA) or serum (PBS pH 7,2 with 2mM EDTA and 0,5% BSA) and dead cells are removed prior magnetic labelling.

For MACS separation, the buffer is degassed by applying vacuum, preferentially with buffer at room temperature.

The magnetic labelling is performed in the refrigerator at a temperature between 6° and 12° C for 15 minutes.

Clumps are removed by passing the cells through a nylon mesh (30 µm) or filter (Miltenyi Biotec GmbH, Bergisch Gladbach, Germany; Order No. 414-07).

For the pre-separation a filter is wetted by vigorously pipetting 500 µl of resuspension buffer in the reservoir prior the filter process and the effluent is discarded. Subsequently a minimum of 500 µl cell suspension with 10⁸ runs through the filter, which is washed 2 to 3 times with 500 µl buffer.

### Depletion of unwanted white blood cells.

After the filter separation the cells are counted and washed in an appropriate buffer.

### Magnetic separation

For the magnetic activated cell sorting the MidiMACS system is used (Miltenyi Biotec GmbH, Bergisch Gladbach, Germany; Order No. 423-01) including the LD-Separation column order No. 429-01.

After washing the column with 3 ml of buffer the magnetic labelled cells suspended in buffer (500 µl buffer/10⁸ total cells) is passed through the column. The effluent is collected as the positive fraction. This step can be repeated. The effluents are compiled and centrifuged with 200 x g for 10 minutes. The pellet is resuspended in 1,5 ml PBS + 0,5 % BSA for further procedures.

### Enrichment of fetal erythrocytes

The aforementioned MideMACS system is operated for the enrichment of fetal erythrocytes by using Glycopherin A (GPA; order No. 422-01) and CD (order No.462-01) conjugated MicroBeads and the buffer system as described under MACS depletion (order No. 424-01).

The cell suspension is allowing to pass the column. Whereas the effluent is discarded the cells having remained within the column are firmly flushed out and collected as the positive fraction.

### 6. Delivery of molecular beacons into the target cells

Enriched fetal cells are washed with HEPES (145 mM NaCI, 5mM KCI, 1mM MgCl₂, 1mM CaCl₂, 10 mM glucose, 10 mM HEPES, pH 7.4), and precipitated by centrifugation. 2 µg of the fluorescent molecular beacons are dissolved in 2 µl of water and mixed with 9 µl of liposomes comprising 0.4 mg of N,N,N',N'-tetramethyl-N-N'-bis(2-hydroxyethyl)-2,3-dioleoyloxy-1,4-butanediammonium iodide and 0.3 mg of L-dioleoyl phosphatidylehtanolamine suspended in 400 µl of nuclease free water (Tfx-50 Reagent, Promega). The mixture is vortexed gently and incubated for 10 min at room temperature. Subsequnetly the cells are washed in HEPES.

### 7. HbF-PE (phycoerithrin) and DAPI staining - Flow cytometric Protocol

### Stain Procedure:

After counting the amount of red blood cells (RBC) within the sample 2,5 x 10⁷ cells are added to 1 ml of cold 0,05 % Glutaraldehyde for 10 min at room temperature. This mixture is washed three times with 2 ml cold PBS- 0,1 % BSA (pH 7,4). Subsequently, the cells are resusupended and incubated in 0,5 ml 0,1 % Triton X 100 for 3-5 minutes at room temperature and subsequently washed in 2 ml of PBS-0,1 % BSA. Afterwards the cells are taken in 0,5 ml PBS- 0,1 % BSA by vortexing or gentle pippeting.

10 µl of this suspension are added to 10 µl antibody (Caltaq Laboratories Burlingame, CA 94010, Product code: MHFH04; Gamma chain antibody) and 70 µl of PBS-0,1 % BSA and incubated at room temperature in the dark for 15 minutes. Two washing steps with 2 ml PBS-0,1 % BSA each are following.

The pellet is suspended in 500µl PBS-0,1 % BSA and 1ml DAPI staining solution (PARTEC, Germany) is added. The incubation takes place for 10 minutes at romm temperature. The pellet is washed twice with 2 ml PBS-0,1 % BSA and finally resuspended by vortex in 0,5 ml 1 % formaldehyde. The cells are stored in tubes in the dark in the refrigerator.

In order to minimise false positive signals an unspecific Phycoerithrin (PE)-stained antibody is used as a negative control during the cytometric detection.

### 8. Cell separation

For cell separation the PASIII - system (PARTEC Germany), is used applying a 488 nm argon laser and an ultraviolet lamp to distinguish different fluorescent dyes within the cells.

### 9. FISH

### Preparation of chromosome or interphase slides

After the cell separation molecular beacon are used to identify fetal cells in flow cytometry and cytogenetic slides with chromosome or nuclear suspension are prepared for fluorescens in situ hybridisation.
(Subcellular Fraction, A practical approach, Edited by J.M.Graham and D. Rickwood; IRL Press: page 79, Protocol 3. Isolation of metaphase chromosomes; page 75, Protocol 1. Purification of cell nuclei from soft tissue).

In order to prepare slides one drop of chromosome or cell nuclear suspension is placed on a very clean glass slide. Slides are rinsed in 2 x SSC, pH 7.0 for 5 min. An incubation for at least 30 min at 37°C in 2 x SSC containing 100 µg/ml RNase A follows. Subsequently the washing of slides is performed for 3 x 5 min in 2 x SSC, pH 7.0 . RNase treatment serves to remove endogenous RNA which may hybridize with homologous probe DNA sequences, forming RNA-DNA hybrids. This improves the signal to noise ratio in hybridization to DNA targets.

The incubation of slides is conducted with pepsin for 10 min at 37°C in 0.05% (w/v) pepsin powder in 0.01 N HCI. Slides are rinsed for 2 x 5 min in 1 x PBS pH 7,3 and then 2 x 5 min in 1 x PBS containing 50 mM MgCl₂. Fixation of slides is achieved with 1% formaldehyde, 1 x PBS, 50 mM MgCl₂ for 10-60 min at room temperature. Slides are thoroughly washed in PBS. Pepsin and other proteases (i.e., proteinase K or pronase) increase accessibility by digesting chromosomal protein that package the target DNA. Since metaphases and nuclei have the tendency to come off the slides after prolonged protease treatment, refixation of the slides with formaldehyde is used.

Dehydration of slides in an ethanol series (70%, 85%, 100%) is performed for 5 min each and air-dry. Then 200 µl of denaturation solution, consisting of 70% deionized formamide and 2 x SSC , is placed on each slide and cover with a coverslip. The slide is placed in 90°C oven for approximately 60 sec. The coverslip is shacked off and immediately put in ice-cold 70% ethanol. Dehydration is performed again in an ice-cold ethanol series (70%, 85%, 100%). After air-drying, the slides are ready for in situ hybridization.

### Preparation and labelling of probe DNA

The DNA template can be supercoiled or linear. After nicking the DNA with DNase I, the 5'-3' exonuclease activity of DNA polymerase removes nucleotides and the DNA polymerase activity replaces the excised nucleotides with dNTPs from the reaction mixture including the labeled nucleotide. The procedures for incorporating biotin, digoxigenin or fluorochromes are nearly identical. The final size of the nick-translated probe DNA fragments is very important. Labeled probe sequences should not be larger than 500 bp because of difficulty penetrating the specimen and a tendency to stick non-specifically to both the glass and the cellular material, resulting in high background which obscures the signal. Sequences shorter than 100 bp do not hybridize efficiently under routine conditions of stringency.

DNA probes are mixed on ice, in a microcentrifuge tube:

| | |
|---|---|
| DNA (in liquid format) | 1 µg |
| 10 x nick-translation buffer (Tris/HCl 1M, pH 8; MgCl₂ 1M; BSA 10%) | 5 µl |
| 0.1 M-mercaptoethanol | 5 µl |
| 10 x cold dNTP solution (0.5 mM each of dATP, dCTP, dGTP) | 5 µl |
| 10 x 1:3 labeled dUTP/cold dTTP solution (0.125 mM labeled dUTP, i.e. biotin-16-dUTP or digoxigenin-11-dUTP, and 0.375 mM dTTP) | 5 µl |

A labeling density of one modified nucleotide at approximately every 20-25^{th} position in the nick-translated DNA is optimal for most FISH experiments.
10 x DNase I 5 µl
(DNase stock solution with an activity of 10 U/µl is diluted 1:3000-1:4000 with double-distilled H₂O)Optimal DNase concentration must be determined by titration, as the size distribution of the probe depends on the amount of enzyme added. Each new stock of DNase I should be tested to determine the appropriate working concentration.
DNA polymerase I (5 U/µl) 1.3 µl
DdH₂O is added as needed to achieve final reaction volume of 50 µl

If larger amounts of DNA (up to 5 µg) need to be labelled, the reaction volumes can be scaled up to 250 µl.

The reaction mixture is incubated for 2 h at 15°C. For optimal incorporation the time of incubation should at least be 2 h, as the initial incorporation rate of modified nucleotides is less than that of unsubstituted dNTP. The size of the nick-translated DNA fragments is checked on a 1.5% agarose gel, along with suitable size markers (0.1-1 kb range). If the DNA fragments are still too large, a second aliquot of DNase (optional) is added and incubated for another 30-60 min at 15°C. When the majority of the fragments are between 100 bp and 500 bp in size, termination of the reaction follows by adding stop buffer (1.25 µl 0.5 M EDTA and 0.5 µl 10% SDS) to the 50 µl of nick-translation mixture. The tube is heated to 68°C for 10 min.

For large-insert clones probes, simple ethanol precipitation is sufficient. For small single-copy DNA probes, purification by centrifugation through Sephadex G-50 or commercially available columns is recommended to remove unincorporated haptenized or fluorescent nucleotides.

In the indirect labelling method hapten reporter molecules, i.e. biotin- or digoxigenin-dUTP, being introduced into the DNA probe, are detected by affinity cytochemistry after the hybridization reaction. Fluorescent (strept)avidin molecules bind specifically to biotin-labeled probe-target hybrids. Fluorescent anti-digoxigenin antibodies are used to detect digoxigenated probes. The fluorescence signals of indirectly labeled probes may be up to tenfold brighter than those produced by direct methods. In addition, small hapten molecules such as biotin are efficiently incorporated into DNA than most fluorescent dNTPs.

### In situ hybridisation (FISH PROTOCOL)

Since interspersed repeats are present in every 5 kb of genomic DNA and most large-insert clones contain a significant number of repetitive elements that can cross-hybridize with closely related repeats throughout the entire genome. Consequently, these probes will not only hybridize to their specific target DNA sequences but to all chromosomes, yielding ambiguous FISH signals. To prevent such unspecific hybridization, repetitive DNA sequences in the probe DNA are blocked by prehybridization with unlabelled total genomic DNA or repetitive cot-1 DNA. The following is a detailed protocol for suppression hybridization that has been optimized for the fluorescent detection of single-copy DNA sequences.

### Hybridization Mixture

Nick-translated DNA probes have a final concentration of approximately 20 ng/µl (in nick-translation buffer). First, differentially labeled probes which are to be hybridized on human specimen are precipitated together with an approximately 50-fold excess of cot-1 competitor DNA (Gibco BRL) or a 500-fold excess of fragmented (100-500 bp) total genomic DNA, and a 50-fold excess of fragmented salmon sperm DNA which is used as a carrier. The optimal DNA concentrations in the hybridization mixture (30 µl for a whole slide) depend mainly on the probe types. For large-insert clones, 10-20 ng/µl in the hybridization mixture (or 300-600 ng per slide) are recommended.

Commercially available large insert clones (Vythis, USA) for specific human chromosomes (21 and Y) are used.

In the following, two different BAC-DNA probes are hybridized together on the same slide for identification of male foetal cells that are affected by trisomie 21.

| Stock | Amount required | |
|---|---|---|
| Biotinylated (nick-translated) BAC # 21 | 20 ng/µl | 20 µl (400 ng) |
| Digoxigenated (nick-translated) BAC# Y | 100 ng/µl | 2 µl (200 ng) |
| Cot-1 DNA (Gibco BRL) | 1 µg/µl | 30 µl (30 µg) |
| Salmon sperm DNA | 10 µg/µl | 3 µl (30 µg) |

The DNA is precipitated with 1/20 volume 3 M Naacetate and 2 volumes 100% EtOH (p.a.) in a microcentrifuge tube. The sample is mixed well and uncubated at least 30 min at -70°C or overnight at -20°C. The precipitation is done in a centrifuge spinning for 30 min at 15,000 rpm at 4°C. The supernatant is discarded and the pellet are dried in a vacuum system or alternatively at 37°C in a heating block.

The DNA probe is resuspended with 15 µl of deionized formamide and shaken at 37°C for at least 15 min to resuspend the probe DNA. Then an equal volume of 2 x hybridization mixture (20% dextran sulfate, 4 x SSC) is added followed by vigorously shaking for at least 15 min.

Conditions of stringency are 50% formamide, 10% dextran sulfate, 2 x SSC in the hybridization mixture. Denaturation of hybridization mixture (containing the probe DNA) is performed at 80°C for 10 min. Probes are centrifuged for 2-5 sec in order to pellet the condensed water.

To allow reannealing of repetitive DNA fractions, the tube with the denatured competitor and probe DNA is incubated at 37°C for at least 15 min. DNA probes (30 µl) are placed on the denatured slide and cover with a coverslip. The edges are sealed with rubber-cement and incubated overnight (or longer) at 37°C in a moist chamber.

30 µl of denatured and pre-annealed DNA probe mixture is pipetted on the prewarmed slide (15 µl for half a slide). The slide is covered with a coverslip without air bubbles and sealed with rubber cement. Hybridisation is done overnight in a moist chamber at 37°C.

Immunocytochemical detection of probe DNA hybridised on chromosome or cell nuclei

Washing of slides follows for 3 x 5 min at 42°C in 50% formamide, 2 x SSC and then 2 x 5 min at 60°C in 0.1 x SSC. The washing solutions are pre-warmed in different water baths. The Coplin jar with slides and pre-warmed washing solution are agitated on a platform shaker.

The probe DNA hybridizes not only to the target DNA but also non-specifically to sequences which bear partial homology to the probe sequence. Since such non-specific hybrids are less stable than perfectly matched DNA hybrids, they can be dissociated by post-hybridization washes of various stringencies.

Secondary incubations and washes are required for the visualization of nick-translated haptenized DNA probes. Prior to incubation with secondary reagents, slides are blocked by treating with 5% bovine serum albumin in 4 x SSC, 0.1% Tween 20. Incubation of slides in a Coplin jar with fresh blocking solution for at least 30 min at 37°C follows. Blocking prevents unspecific antibody binding and reduces background.

Fluorescent (strept)avidin and anti-digoxigenin antibodies, i.e. FITC-avidin and Cy3-conjugated anti-digoxigenin, are diluted 1:800 and 1:200, respectively (or according to recommendations of the supplier) in 1% BSA, 0.1% Tween 20, 4 x SSC. Cover slides with 200 µl of detection solution and a coverslip. Incubation is performed in a moist chamber in the dark at 37°C for 30 min. Coverslips are shaken off the and washing slides 3 x 5 min in a Coplin jar with 0.1% Tween 20, 4 x SSC at 42°C. To visualize chromosomes and cell nuclei, counterstain slides for 1-2 min with 1 µg/ml DAPI (4',6-diamidino-2-phenylindole) in 2 x SSC. Washing of the slides is performed thoroughly with distilled water, air-dry and then mount them with antifade mounting medium . DABCO retards fading of the fluorescence after exposure of the slides to fluorescence light.

Large-insert clones produce specific fluorescence signals, which are clearly visible by eye through the microscope. Digoxigenated DNA probes binding fluorescent mouse anti-digoxigenin antibody are amplified with a second layer of fluorescent rabbit anti-mouse antibodies and, if necessary, with a third layer of fluorescent goat anti-rabbit antibodies.

Because of the high sensitivity of digital cameras, use of electronic image enhancement is arguably preferred over immunocytochemical signal amplification. Specialized FISH software allows the conversion of the plain DAPI fluorescence of a chromosome into a G-like banding pattern. We used ISIS 3 Fluorescens picture system and a HAMAMATSU Digital camera system (Metasystems Germany).

### Annex to the application documents - subsequently filed sequences listing

SEQUENCE LISTING

<110> Praenadia GmbH

<120> A method for direct genetic analysis of target cells by
using fluorescence probes

<130> 42 850 K

<140> 00115268.5-2116
<141> 2000-07-14

<160> 9

<170> PatentIn Ver. 2.1

<210> 1
<211> 20
<212> DNA
<213> Artificial Sequence

<220>
<223> Description of Artificial Sequence: PCR primer for
beta-actin, downstream

<400> 1
gttggcgtac aggtctttgc 20

<210> 2
<211> 15
<212> DNA
<213> Artificial Sequence

<220>
<223> Description of Artificial Sequence: PCR primer for
beta-actin, upstream

<400> 2
ggccacggct gcttc 15

<210> 3
<211> 26
<212> DNA
<213> Artificial Sequence

<220>
<223> Description of Artificial Sequence: PCR primer for
HBG1, downstream

<400> 3
aagacagcac cttcttgcca tgtgcc 26

<210> 4
<211> 24
<212> DNA
<213> Artificial Sequence

<220>
<223> Description of Artificial Sequence: PCR primer for
HBG1, upstream

<400> 4
tttcacagag gaggacaagg ctac 24

<210> 5
<211> 28
<212> DNA
<213> Artificial Sequence

<220>
<223> Description of Artificial Sequence: molecular
beacon for beta-actin

<400> 5
gctcgtccat gcccaggaag gaacgagc 28

<210> 6
<211> 36
<212> DNA
<213> Artificial Sequence

<220>
<223> Description of Artificial Sequence: molecular
beacon for Zfy

<400> 6
gcgagtagtc cacttttctt tacattttat tctcgc 36

<210> 7
<211> 35
<212> DNA
<213> Artificial Sequence

<220>
<223> Description of Artificial Sequence: molecular
beacon for HBG1

<400> 7
gcgagaccca gaggttcttt gacagctttg ctcgc 35

<210> 8
<211> 37
<212> DNA
<213> Artificial Sequence

<220>
<223> Description of Artificial Sequence: molecular
beacon for CFTR exon 10

<400> 8
gcgagaaaga aaatatcatc tttggtgttt ccctcgc 37

<210> 9
<211> 34
<212> DNA
<213> Artificial Sequence

<220>
<223> Description of Artificial Sequence: molecular
beacon for CFTR exon 10

<400> 9
gcgagaaaga aaatatcttt ggtgtttccc tcgc 34

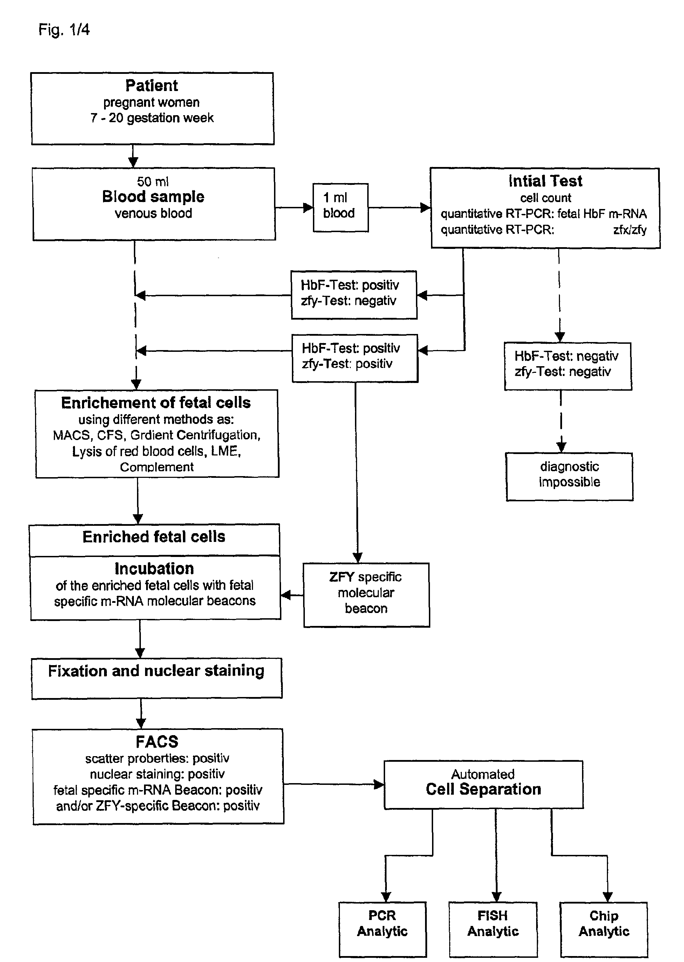

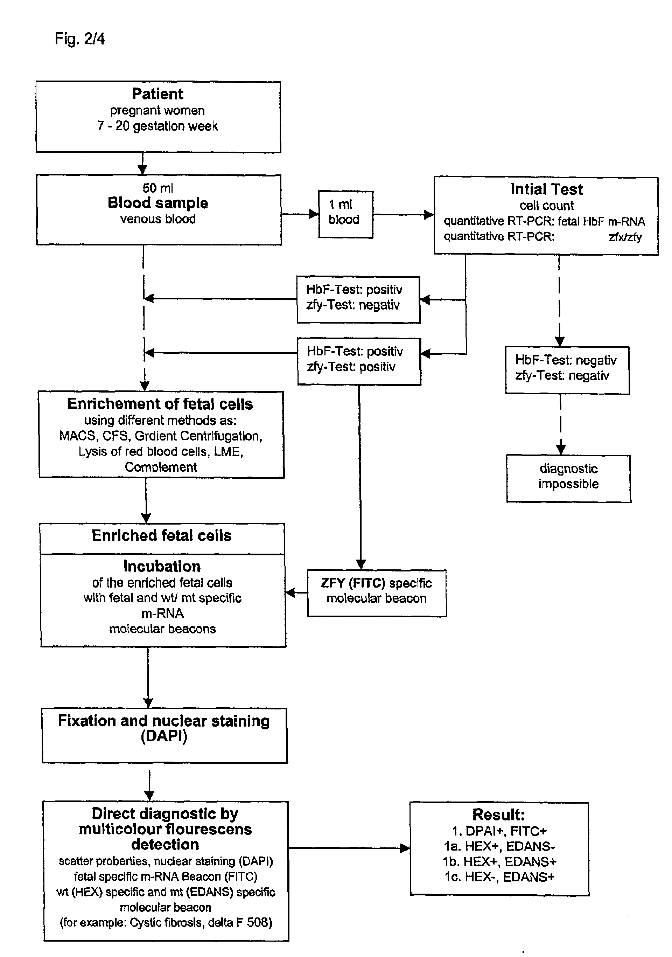

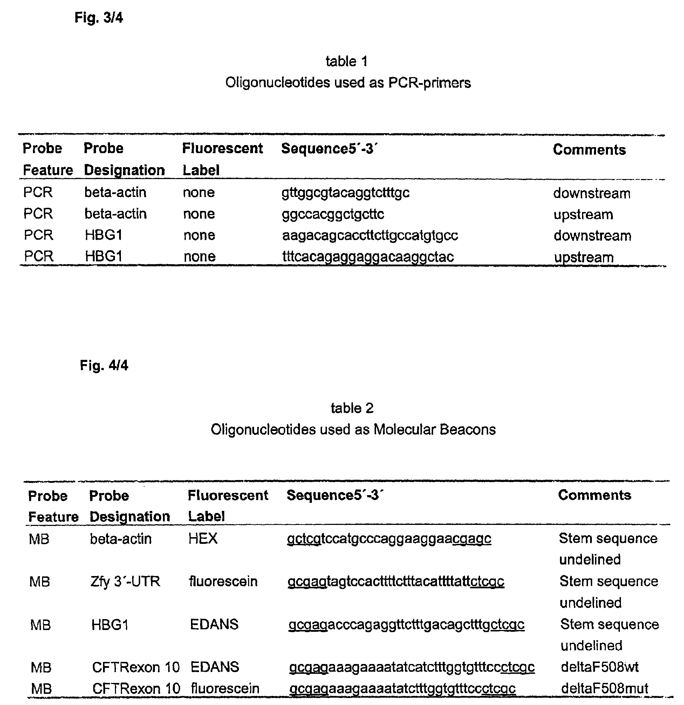

## Claims

1. Method for identifying a target cell comprising the following steps:
- in situ hybridising of a target sequence in a target cell with a complementary labelled sequence and
- cell identification of the target cell by the aid of detecting hybridised sequence by flow cytometry.

2. Method for direct genetic analysis comprising the following steps:
- in situ hybridising of a target sequence in a target cell with a complementary labelled sequence and
- cell identification of the target cell by the aid of detecting hybridised sequence by flow cytometry.

3. Method according to claim 1 or 2 **characterised in** the labelled sequences being molecular beacons.

4. Method according to one of the above claims **characterised in** fluorescence activated cell sorting.

5. Method according to one of the above claims **characterised in** at least one part of the labelled sequences being directed against a target fetal specific mRNA.

6. Method according to claim 5 whereas the fetal specific-mRNA is selected from the group consisting of fetal hemoglobin mRNA, cytokeratin mRNA, β-subunit of chorionic gonadotropin mRNA, chorionic somatomammotropin mRNA, pregnancy-specific glycoprotein mRNA, α-fetoprotein mRNA and transferrin receptor mRNA.

7. Method according to claim 1 to 4 whereas at least one part of the labelled sequence is complementary to a target sequence selected from the group consisting of target specific DNA, viral nucleic acids or bacterial nucleic acids, or parts thereof.

8. Method according to one of the above claims comprising at least one step of negative selection of non-target cells applying a method selected of the group consisting of density gradient centrifugation, lysis by hypotonic shock, destruction by complement system, destruction by lysomotropic agent, charge flow separation and magnetic activated cell sorting.

9. Method according to one of the above claims comprising at least one step of positive selection of target cells applying a method selected of the group consisting of charge flow separation or magnetic activated cell sorting.

10. Method according to one of the above claims including the step of intracellular staining or staining of surface markers of the target cells.

11. Method according to claim 10 **characterised in** antibody staining.

12. Use of a method according to one of the above claims for the distinction of fetal and maternal cells.

13. Use of a method according to one of the above claims for prenatal diagnosis.

14. Use of a method according to one of the above claims for the detection of cystic fibrose.

15. Use of a method according to one of the above claims for the detection of chromosomal aberration.

16. Use of a method according to one of the above claims for the detection of trisomy 21.

17. Method for quantifying fetal mRNA within a maternal blood sample comprising the following steps:
- amplifying fetal cell specific mRNA;
- synthesising labelled sequences comprising a sequence being complementary or partly complementary to said amplified fetal cell specific mRNA;
- hybridising said labelled sequence with said amplified fetal cell specific mRNA and
- detecting the hybridised labelled complementary sequence.

18. Method according to claim 17 **characterised in** at least one part of the labelled complementary sequences being molecular beacons.

19. Method according to one of the above claims **characterised in** the molecular beacon comprising at least one of the sequences enlisted in table 2.

20. Method according to one of the claims 17 to 19 whereas a primer for the amplification comprises one of the sequences enlisted in table 1.

21. Kit for cell identification and / or genetic analysis comprising a test tube with at least one compartment containing molecular beacons.

22. Kit according to claim 21 with at least two compartments whereas each of the compartments comprises a different media.

23. Test tube according to claim 21 or 22 **characterised in** a membrane separating the compartments one from the other.

24. Method for processing probes within a test tube with at least two compartments comprising the following steps:
- treatment of the probe in the first compartment;
- providing access for said probe to the second compartment and
- treatment of said probe in the second compartment.

25. Method according to claim 24 **characterised in** one of the treatments being the hybridising of the probe with molecular beacons.
